# FASCICULE DE BREVET EUROPEEN

(11) **EP 1 363 699 B1**
(45) Date de publication et mention de la délivrance du brevet: **26.04.2006**
(21) Numéro de dépôt: 02714270.2
(22) Date de dépôt: 28.02.2002
(51) Int. Cl.: A61N 1/05, A61N 1/04, A61B 5/0408

(54) **STRUCTURE D'ELECTRODES IMPLANTABLE**
IMPLANTIERBARE ELEKTRODENSTRUKTUR
IMPLANTABLE ELECTRODE STRUCTURE

(30) Priorité: 28.02.2001 FR 0102727
(43) Date de publication de la demande: 26.11.2003
(73) Titulaire: Microvitae Technologies, 13790 Peynier (FR)
(72) Inventeur: HERVE, Thierry, 38100 Grenoble (FR)
(74) Mandataire: de Beaumont, Michel
(86) Numéro de dépôt international: PCT/FR2002/000732
(87) Numéro de publication internationale: WO 2002/068041

(56) Documents cités:
- WO-A-99/49934
- US-A- 4 261 372
- US-A- 5 873 901
- US-A- 5 897 583
- US-A- 5 919 220
- US-A- 6 151 526

## Description

La présente invention concerne une structure portant des électrodes susceptibles de détecter l'activité électrique d'un organe, comme un nerf ou le coeur. Document US-A-6 151 526 décrit une électrode pour stimulation cochlé aire avec des électrodes qui sont situées sur le côté opposé à l'excroissance 205.

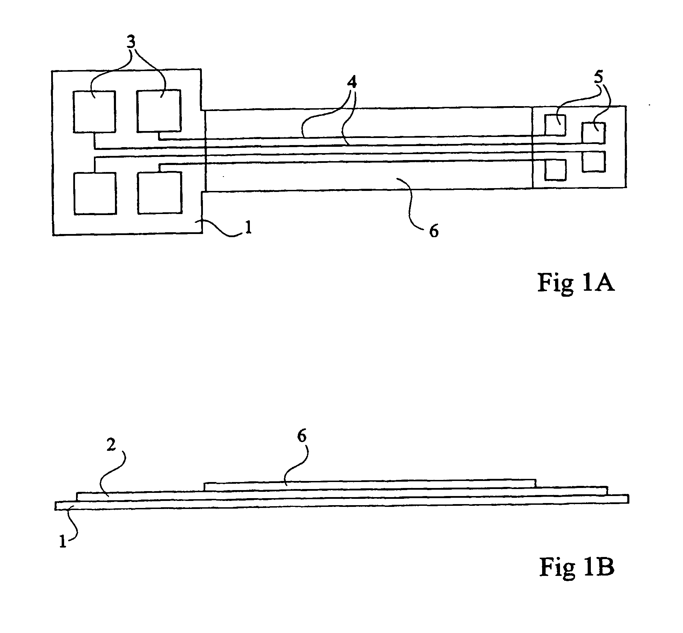

La figure 1A décrit une structure connue de ce type. La figure 1B représente la structure de la figure 1A en coupe. La structure comporte un film support 1 en un matériau souple isolant comme du polyimide. Sur ce film, est déposée une couche conductrice 2 gravée de façon à délimiter des électrodes 3, reliées par des pistes conductrices 4 à des plots de connexion 5. A l'exception des électrodes et des plots, l'ensemble est recouvert d'un film isolant 6. La structure obtenue est souple et a une épaisseur très faible, de l'ordre de quelques micromètres. Typiquement, les électrodes 3 ont une taille de quelques micromètres, et la structure a une longueur de quelques centimètres.

Lors de son utilisation, la structure est insérée sur ou au voisinage de l'organe dont l'activité électrique doit être mesurée. Lors de l'insertion de la structure, la manipulation de la structure est peu aisée du fait de sa souplesse. Si la structure est manipulée avec des pinces, elle peut être endommagée. Lors de son insertion, elle peut se déformer et parvenir difficilement à l'endroit souhaité. En outre, une fois mise en place, la structure de l'art antérieur garde difficilement une forme déterminée quelconque. Elle a tendance à glisser et à s'écarter de la position où elle a été placée.

Un objet de la présente invention est de réaliser une structure à électrodes du type susmentionné permettant une manipulation aisée et une insertion facilitée.

Un autre objet de la présente invention est de réaliser une telle structure gardant facilement une forme quelconque souhaitée.

Un autre objet de la présente invention est de réaliser une structure qui ne glisse pas de l'endroit où elle a été placée.

Pour atteindre ces objets ainsi que d'autres, la présente invention prévoit une structure comportant un film support isolant souple et des électrodes propres à mesurer l'activité électrique d'un milieu physiologique ou à le stimuler, l'ensemble formé par ledit film et lesdites électrodes étant d'une épaisseur inférieure à 50 micromètres. La structure comporte, du côté des électrodes, au moins un élément en matière isolante en saillie par rapport à la surface de la structure, l'épaisseur dudit élément étant égale à au moins 20 micromètres.

Selon un mode de réalisation de la présente invention, les électrodes comportent des trous traversant la structure.

Selon un mode de réalisation de la présente invention, l'élément en saillie a la forme d'un ergot vertical propre à coopérer avec une ouverture de la structure.

Selon un mode de réalisation de la présente invention, l'élément en saillie a la forme d'un anneau continu ou discontinu et entoure les électrodes.

Selon un mode de réalisation de la présente invention, l'élément en saillie a la forme de plusieurs ergots verticaux placés au niveau de la partie de la structure qui comporte les électrodes.

Selon un mode de réalisation de la présente invention, l'élément en saillie a la forme d'une barre rigide longitudinale.

Selon un mode de réalisation de la présente invention, l'élément en saillie a la forme d'un ergot latéral.

Selon un mode de réalisation de la présente invention, la structure comporte plusieurs éléments en saillie dont le nombre et la répartition sont variables, de sorte que la rigidité de la structure est contrôlée.

Selon un mode de réalisation de la présente invention, la structure comporte des ouvertures dans sa partie isolante.

La présente invention prévoit aussi un ensemble comprenant une structure ci-dessus et un dispositif rigide et/ou déformable, la structure étant solidaire dudit dispositif.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
les figures 1A et 1B, précédemment décrites, représentent des structures à électrodes de l'art antérieur ;
la figure 2 représente un premier mode de réalisation de la présente invention ;
la figure 3 représente un deuxième mode de réalisation de la présente invention ;
les figures 4A et 4B représentent respectivement un troisième mode de réalisation de la présente invention et une utilisation possible de celui-ci ;
les figures 5A et 5B représentent respectivement un quatrième et un cinquième mode de réalisation de la présente invention ; et
les figures 6A et 6B illustrent des étapes permettant de réaliser des structures selon la présente invention.

Dans les figures, de mêmes signes références représentent des éléments identiques ou ayant la même fonction. Les échelles n'ont pas été respectées, notamment en ce qui concerne la dimension verticale.

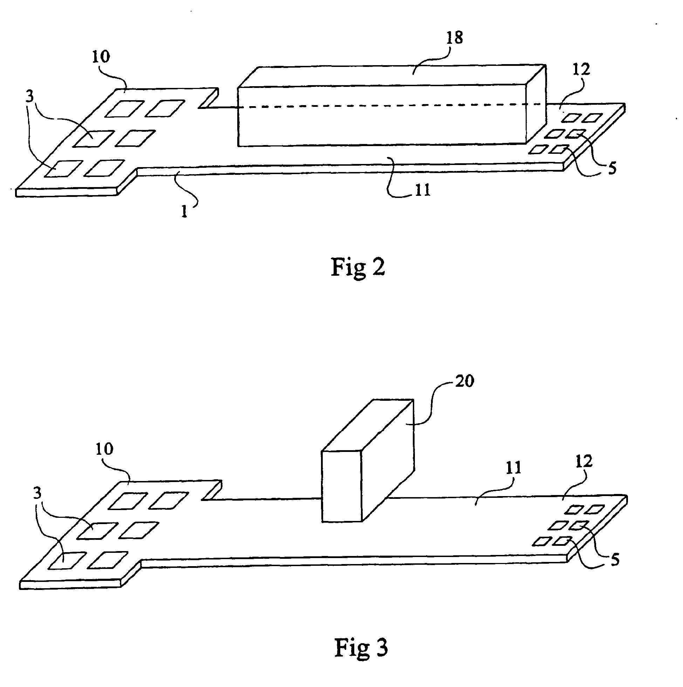

En figure 2, un film 1 d'une structure selon un premier mode de réalisation de la présente invention comporte une partie de tête 10 portant des électrodes 3, une partie intermédiaire 11 et une partie terminale 12 portant des plots 5. Par souci de simplicité, les pistes de connexion entre les électrodes et les plots n'ont pas été représentées. Sur la partie intermédiaire 11, se trouve une barre longitudinale parallélépipédique 18. La barre 18 représente un élément tridimensionnel formant un relief en saillie par rapport à la surface de la structure, du même côté que les électrodes. La barre 18 a une hauteur variable par rapport à l'épaisseur du film support et de la couche conductrice formant les électrodes. Alors que le film support, de même que la couche formant les électrodes, a une épaisseur typique de quelques micromètres seulement, la hauteur de la barre 18 peut aller de 20 micromètres environ à 500 micromètres ou plus. Dans un exemple pratique de réalisation, la hauteur de la barre 18 a été choisie égale à 100 micromètres.

La barre 18 rend la partie intermédiaire 11 plus rigide et peut servir de guide. Par exemple, elle peut s'insérer dans une glissière d'un élément rigide utilisé pour insérer la structure. La barre 18 peut être aussi fixée à l'extrémité d'un dispositif quelconque, par exemple de type endoscope ou cadre stéréostatique. Ainsi, la structure peut être placée avec une précision submillimétrique. Cette précision du positionnement de la structure, possible avec la présente invention, peut avoir un intérêt majeur dans certains cas, comme lorsque la structure est placée dans le cerveau. La barre 18 peut être aussi fixée sur un dispositif destiné à rester dans le corps. La barre 18 peut aussi être prise par une pince, facilitant ainsi l'insertion de la structure ou sa manipulation en cours d'intervention.

Dans une variante de réalisation non représentée, la barre 18 se réduit à un simple parallélépipède rectangle de section carrée et constitue un ergot vertical disposé par exemple au centre ou vers l'avant de la structure. Cet ergot pourra être saisi par une pince et faciliter la manipulation de la structure.

La figure 3 représente un deuxième mode de réalisation de la présente invention. La structure de la figure 3 est en grande partie semblable à celle de la figure 2. Elle comporte aussi un élément tridimensionnel en saillie par rapport à la surface de la structure. Ici, l'élément en saillie a la forme d'un ergot latéral 20 situé au niveau de la partie intermédiaire 11 de la structure. L'ergot 20 a une hauteur qui, comme la barre 18 de la figure 2, se situe dans une plage allant de 20 micromètres environ à 500 micromètres ou plus.

L'ergot 20 peut servir à la manipulation et peut aisément être saisi par une pince. L'ergot 20 peut servir d'autres buts que la manipulation. Par exemple, il peut servir de point d'arrêt à une glissière sur laquelle est insérée la partie arrière de la structure. L'ergot 20 peut ainsi coopérer avec la barre 18 de la figure 2, et stopper en translation une glissière dans laquelle la barre 18 est insérée. En figure 3, l'ergot 20 est disposé au niveau de la partie intermédiaire 11 de la structure. Bien entendu, il peut se trouver au niveau d'une partie quelconque de la structure, par exemple au niveau de la partie de tête 10.

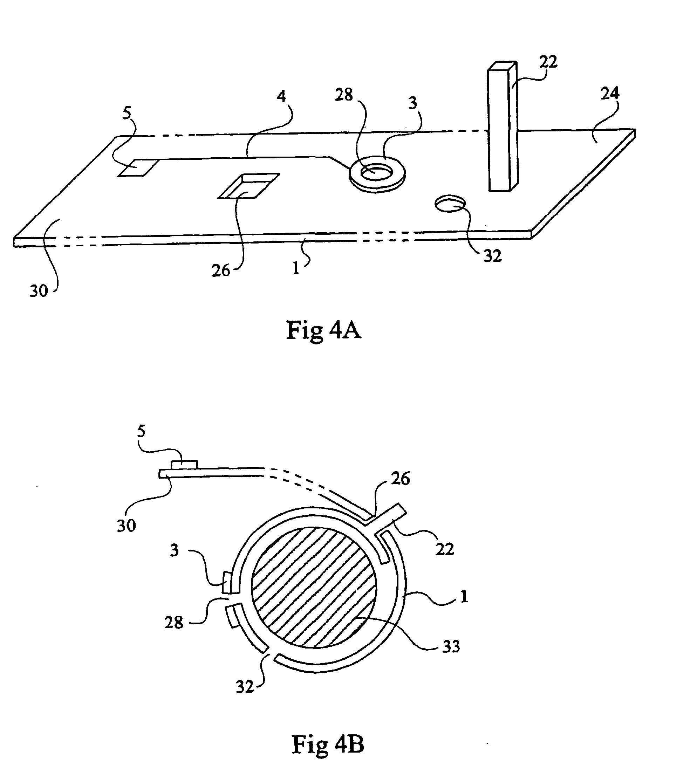

La figure 4A représente un autre mode de réalisation d'une structure selon la présente invention. Comme dans les modes de réalisation précédents, la structure comporte un élément en saillie, ici sous la forme d'un ergot vertical 22, de forme parallélépipédique et de hauteur allant de 20 micromètres environ à 500 micromètres ou plus. L'ergot 22 est situé près d'une extrémité 24 de la structure. La structure comporte également une ouverture 26 traversant la structure de part en part. L'ouverture 26 a une taille telle que l'ergot 22 peut s'insérer dans l'ouverture 26. Entre l'ouverture 26 et l'ergot 22 sont disposées des électrodes 3, dont une seulement est représentée. Chaque électrode 3 est percée d'un trou perforant 28 et est reliée par une piste conductrice 4 à un plot 5. Les plots 5 sont situés à une extrémité 30 de la structure, opposée à l'extrémité 24. La structure peut comporter aussi des trous perforants 32 (un seul est représenté) entre l'ouverture 26 et l'ergot 22.

La figure 4B représente un mode d'utilisation possible de la structure de la figure 4A. En figure 4B, la partie hachurée représente un nerf 33 en coupe. La structure est enroulée autour du nerf et l'ergot 22 est inséré dans l'ouverture 26. La distance entre l'ouverture 26 et l'ergot 22 est choisie de sorte que la structure ne serre pas trop le nerf, tout en permettant une mesure adéquate de son activité. Les électrodes 3 sont disposées autour du nerf. Comme les électrodes se trouvent sur la surface du film 1 qui ne fait pas contact avec le nerf, le trou 28 qui traverse chaque électrode permet au signal électrique de passer du nerf à l'électrode. D'autres trous 32 peuvent être situés dans la partie isolante de la structure située entre l'ouverture 26 et l'ergot 22. Leur rôle est de laisser passer le liquide physiologique, et de permettre un équilibre de ce liquide de part et d'autre de la structure. Les plots 5, à l'extrémité 30, permettent la connexion à un circuit extérieur.

On notera que les trous 28 des électrodes servent aussi à l'irrigation, comme les trous 32, et que les trous 32 favorisent aussi la conduction électrique. Le rôle d'irrigation dû aux trous 28 et 32 est très important dans certaines applications. Par exemple, dans l'application ci-dessus, un nerf non irrigué perd sa fonctionnalité très rapidement, ce qui n'est pas souhaitable.

On notera aussi que, grâce à la coopération de l'ergot 22 et de l'ouverture 26, la structure est bien fixée autour du nerf et ne glisse pas. Le cas échéant, plusieurs ouvertures 26 peuvent être pratiquées dans la structure, afin d'adapter la structure à différentes tailles de nerfs ou d'organes autour desquels elle est insérée.

Dans une variante, plusieurs ergots 22 disposés à intervalles réguliers ou non peuvent s'insérer chacun dans une ouverture 26 de la structure. La structure peut prendre ainsi une forme complexe particulièrement stable dans le temps, par exemple la forme d'un cylindre, d'une spirale ou d'une hélice.

Dans une variante, la structure comporte un ou plusieurs ergots 22 destinés à s'insérer dans des ouvertures d'un dispositif rigide extérieur à la structure. Le dispositif extérieur peut être non plan et avoir une forme quelconque déterminée.

La structure peut aussi être fixée à un dispositif rigide et/ou déformable dont elle est solidaire. Le dispositif rigide et/ou déformable peut être réalisé à l'aide de matériaux à mémoire de forme et peut être adapté à la concavité particulière d'un organe, par exemple la cochlée.

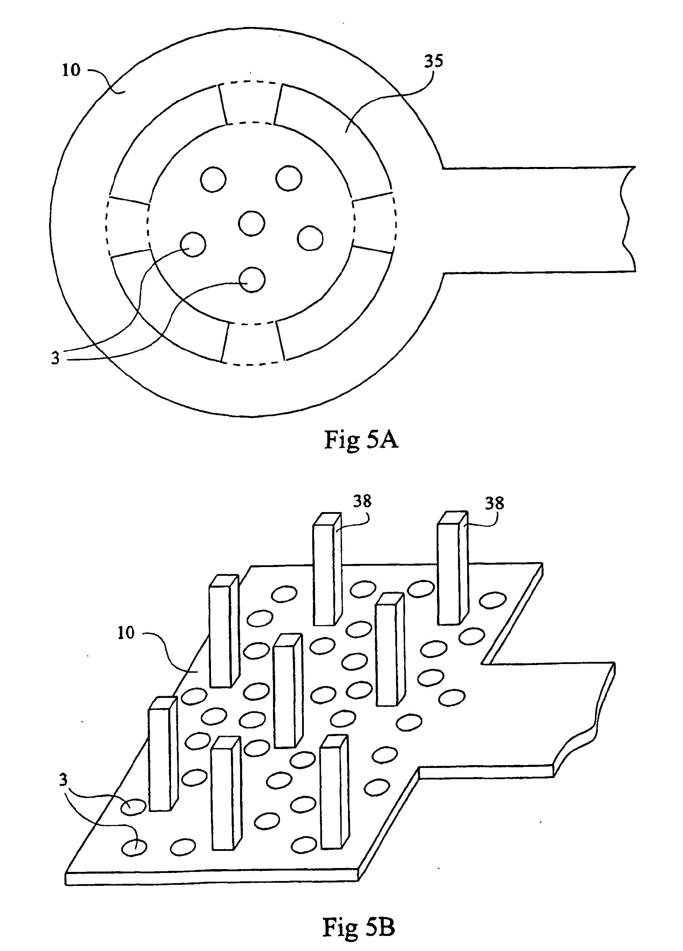

La figure 5A représente un autre mode de réalisation de la présente invention. En figure 5A, seule la partie de tête 10 de la structure est représentée, ici sous la forme d'un disque. Des électrodes 3, ici de forme circulaire, sont disposées dans la partie centrale de la partie 10. Les électrodes 3 sont entourées d'un élément en saillie 35 de forme annulaire. L'élément 35 peut former un anneau continu ou un anneau discontinu, afin de ne pas trop augmenter la rigidité de la partie 10. La hauteur de l'élément 35 est de même ordre de grandeur que la hauteur des éléments en saillie des modes de réalisation précédents. L'élément 35 forme un rebord qui s'oppose au glissement de la partie 10 sur la surface où elle est appliquée.

Aussi, dans de nombreuses applications, on utilise une pâte ou un gel conducteur destiné à favoriser le contact électrique entre les électrodes et l'organe, par exemple la peau. Dans l'art antérieur, la présence d'un gel favorise le glissement de la structure sur l'organe. Aussi, le gel peut quitter la structure et ne plus remplir sa fonction. Dans la présente invention, l'élément 35, qui forme une sorte de cuvette, peut servir de réceptacle au gel, qui est retenu, même dans les cas où l'élément 35 est discontinu.

La figure 5B représente un autre mode de réalisation de la présente invention. La structure de la figure 5B présente les mêmes avantages que la structure de la figure 5A. En figure 5B, la partie de tête 10 comporte un nombre relativement élevé d'ergots 38, de forme comparable aux ergots 22 de la figure 4A. Les ergots 38 permettent, de part l'aspérité qu'ils représentent, un bon accrochage à la surface sur laquelle ils sont appliqués. Lorsque l'on place une pâte ou un gel conducteur sur la partie 10, les ergots 38 retiennent le gel ou la pâte, qui ne quittent pas la surface de la partie 10 de la structure. En figure 5B, les électrodes 3 sont réparties de manière sensiblement uniforme entre les ergots 38.

Un autre avantage des éléments 35 et 38 est que les structures qui les comportent peuvent facilement être insérées dans un vêtement ou un sous-vêtement. Le vêtement et sa position sur le corps doivent permettre le passage de signaux électriques de l'organe testé, par exemple la peau, aux électrodes de la structure. Par exemple, le vêtement sera ouvert ou conducteur au niveau des électrodes, et en contact avec la peau. Grâce aux éléments 35, 38, la structure est particulièrement bien fixée aux fibres du tissu formant le vêtement et reste dans une position stable. Une telle structure, insérée dans un vêtement, permet des mesures en continu, comme une surveillance cardiaque.

Dans un mode de réalisation non représenté de la structure, des éléments en saillie ont simplement pour but de rigidifier la structure. Par exemple, une série d'ergots du type de l'ergot 22 sont disposés le long de la partie intermédiaire 11, la rigidité de la structure étant fonction du nombre et de la répartition des ergots. Ainsi, le fait de disposer des éléments en saillie permet de contrôler localement la rigidité de la structure. Cela est particulièrement intéressant dans des cas où, une fois placée, la structure doit présenter des parties de courbure différente. Ainsi, les parties rectilignes de la structure seront avantageusement relativement rigides, alors que les parties courbes auront une rigidité moindre.

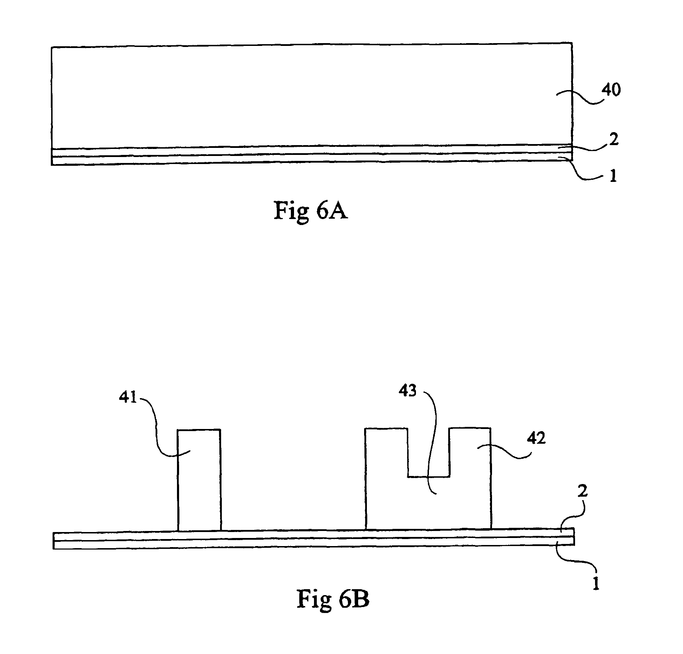

On va maintenant décrire, en relation avec les figures 6A et 6B, un procédé permettant de réaliser des structures selon la présente invention.

En figure 6A, un film support 1 en matière souple isolante, par exemple en polyimide, est recouvert d'une couche conductrice 2. L'ensemble formé par le film 1 et la couche 2 a une épaisseur faible, généralement inférieure à 50 micromètres. La couche conductrice 2 est convenablement gravée pour faire apparaître des électrodes reliées à des plots de contact permettant la connexion à un circuit extérieur. Sur la couche 2 est déposée une couche épaisse 40 de matière isolante, par exemple le même matériau que la couche 1 ou un polymère gravable sélectivement par rapport au matériau de la couche 1. La couche 40 est déposée en une ou plusieurs fois. L'épaisseur de la couche 40 n'est pas critique. Elle peut aller de 20 micromètres environ à 500 micromètres ou plus. Ensuite, la couche 40 est gravée, par exemple par plasma.

La figure 6B représente un exemple possible de structure résultant de la gravure de la couche 40. La couche 40 a été gravée totalement par endroits et de façon incomplète en d'autres endroits. Dans la structure résultante, les couches 1 et 2 demeurent inchangées, et il subsiste, à la surface de la structure, deux éléments tridimensionnels en saillie, 41 et 42. L'élément 41 a une forme parallélépipédique. Il peut se présenter par exemple sous la forme d'une barre verticale s'étendant sur toute la largeur de la structure, ou sous la forme d'un ergot de section par exemple carrée. L'élément 42 a une forme en créneaux et présente une partie 43 incomplètement gravée.

Bien entendu, la présente invention est susceptible de diverses variantes et modifications qui apparaîtront à l'homme de l'art. En particulier, les modes de réalisation décrits dans les figures 2, 3, 4A, 5A et 5B sont des exemples seulement et toute structure à électrodes portant, du côté des électrodes, des éléments en saillie à sa surface fait partie du domaine de la présente invention, quelle que soit la fonction particulière des éléments en saillie.

Les électrodes 3 peuvent être disposées n'importe où à la surface de la structure, de même que les éléments en saillie de la structure.

Les trous 32 décrits en relation avec les figures 4A et 4B peuvent être présents sur toute structure selon la présente invention et à tout emplacement. De même, les électrodes 3 peuvent présenter des trous 28 dans toute structure selon la présente invention.

Bien que la structure de la présente invention ait été décrite comme servant à détecter l'activité électrique d'un organe, toute application de la structure fait partie du domaine de l'invention, comme la stimulation d'un organe à l'aide de signaux électriques fournis par les électrodes 3.

On notera que la structure de la présente invention peut comporter une couche isolante supplémentaire recouvrant l'ensemble constitué par le film 1 et la couche conductrice formant les électrodes, les plots et les pistes entre ceux-ci. Dans ce cas, la couche isolante supplémentaire est une couche mince flexible pouvant être de même épaisseur que le film 1 et ne recouvre pas la structure à l'emplacement des électrodes et des plots. Cette couche isolante supplémentaire correspond à la couche 6 de l'art antérieur. Elle peut résulter du dépôt d'une couche mince sur la structure, ou d'une gravure incomplète de la couche épaisse 40.

## Revendications

1. Structure comportant un film support isolant souple (1) et des électrodes (3) propres à mesurer l'activité électrique d'un milieu physiologique ou à le stimuler, l'ensemble formé par ledit film et lesdites électrodes étant d'une épaisseur inférieure à 50 micromètres, **caractérisée en ce qu'**elle comporte, du même côté que les électrodes, au moins un élément (18, 20, 22, 35, 38) en matière isolante en saillie par rapport à la surface de la structure, l'épaisseur dudit élément étant égale à au moins 20 micromètres.

2. Structure selon la revendication 1, dans laquelle les électrodes (3) comportent des trous (28) traversant la structure.

3. Structure selon l'une quelconque des revendications 1 et 2, dans laquelle l'élément en saillie a la forme d'un ergot vertical (22) propre à coopérer avec une ouverture (26) de la structure.

4. Structure selon l'une quelconque des revendications 1 et 2, dans laquelle l'élément en saillie a la forme d'un anneau continu ou discontinu (35) et entoure les électrodes.

5. Structure selon l'une quelconque des revendications 1 et 2, dans laquelle l'élément en saillie a la forme de plusieurs ergots verticaux (38) placés au niveau de la partie de la structure qui comporte les électrodes.

6. Structure selon l'une quelconque des revendications 1 et 2, dans laquelle l'élément en saillie a la forme d'une barre rigide longitudinale (18).

7. Structure selon l'une quelconque des revendications 1 et 2, dans laquelle l'élément en saillie a la forme d'un ergot latéral (20).

8. Structure selon l'une quelconque des revendications 1 à 7, comportant plusieurs éléments en saillie dont le nombre et la répartition sont variables, de sorte que la rigidité de la structure est contrôlée.

9. Structure selon l'une quelconque des revendications 1 à 8, comportant des ouvertures (32) dans sa partie isolante.

10. Ensemble comprenant une structure selon l'une quelconque des revendications 1 à 9 et un dispositif rigide et/ou déformable, la structure étant solidaire dudit dispositif.

## Patentansprüche

1. Struktur, welche einen biegsamen, isolierenden Tragfilm (1) sowie zur Messung der elektrischen Aktivität eines physiologischen Milieus oder zu dessen Stimulation geeignete Elektroden (3) umfasst, wobei das aus dem genannten Film und den genannten Elektroden gebildete Aggregat eine Dicke kleiner als 50 Mikrometer besitzt, **dadurch gekennzeichnet, dass** die Struktur auf derselben Seite wie die Elektroden wenigstens ein über die Oberfläche der Struktur überstehendes Element (18, 20, 22, 35, 38) aus einem isolierenden Material aufweist, wobei die Dicke des genannten Elements wenigstens gleich 20 Mikrometer ist.

2. Struktur nach Anspruch 1, bei welcher die Elektroden (3) die Struktur durchsetzende Löcher bzw. Öffnungen (28) besitzen.

3. Struktur nach einem der Ansprüche 1 und 2, bei welcher das überstehende Element die Form eines vertikalen Vorsprungs bzw. Fingers (22) besitzt, der mit einer Öffnung (26) der Struktur zusammenwirken kann.

4. Struktur nach einem der Ansprüche 1 und 2, bei welcher das überstehende Element die Form eines kontinuierlichen oder diskontinuierlichen Rings (35) besitzt und die Elektroden umgibt.

5. Struktur nach einem der Ansprüche 1 und 2, bei welcher das überstehende Element die Form mehrerer vertikaler Vorsprünge oder Finger (38) besitzt, die im Bereich des die Elektroden aufweisenden Teils der Struktur angeordnet sind.

6. Struktur nach einem der Ansprüche 1 und 2, bei welcher das überstehende Element die Form eines länglichen starren Balkens bzw. Barren (18) besitzt.

7. Struktur nach einem der Ansprüche 1 und 2, bei welcher das überstehende Element die Form eines seitlich bzw. quer verlaufenden Vorsprungs bzw. Fingers (20) besitzt.

8. Struktur nach einem der Ansprüche 1 bis 7, welche mehrere überstehende Elemente umfasst, deren Zahl und Verteilung variabel sind, derart dass die Starrheit bzw. Steifigkeit der Struktur steuerbar ist.

9. Struktur nach einem der Ansprüche 1 bis 8, welche Öffnungen (32) in ihrem isolierenden Teil aufweist.

10. Aggregat, welches eine Struktur gemäß einem der Ansprüche 1 bis 9 und eine starre und/oder verformbare Vorrichtung umfasst, und wobei die Struktur fest mit der Vorrichtung verbunden ist.

## Claims

1. A structure comprising a flexible isolating support film (1) and electrodes (3) capable of measuring the electric activity of a physiological medium or of stimulating it, the assembly formed by said film and said electrodes having a thickness smaller than 50 micrometers, **characterized in that** it comprises, on the same side as the electrode, at least one element (18, 20, 22, 35, 38) of isolating material protruding from the surface of the structure, the thickness of said element being equal to at least 20 micrometers.

2. The structure of claim 1, wherein the electrodes (3) comprise holes (28) running through the structure.

3. The structure of claim 1 or 2, wherein the protruding element has the shape of a vertical toe (22) capable of cooperating with an opening (26) of the structure.

4. The structure of claim 1 or 2, wherein the protruding element has the shape of a continuous or discontinuous ring (35) and surrounds the electrodes.

5. The structure of claim 1 or 2, wherein the protruding element has the shape of several vertical toes (38) placed at the level of the structure portion which comprises the electrodes.

6. The structure of claim 1 or 2, wherein the protruding element has the shape of a rigid longitudinal bar (18).

7. The structure of claim 1 or 2, wherein the protruding element has the shape of a lateral toe (20).

8. The structure of any of claims 1 to 7, comprising several protruding elements of variable number and distribution, so that the rigidity of the structure is controlled.

9. The structure of any of claims 1 to 8, comprising openings (32) in its isolating portion.

10. An assembly comprising the structure of any of claims 1 to 9 and a rigid and/or deformable device, the structure being integral with said device.
